# EUROPEAN PATENT APPLICATION

(11) **EP 3 034 096 A1**
(43) Date of publication of application: **22.06.2016**
(21) Application number: 14199136.4
(22) Date of filing: 19.12.2014
(51) Int. Cl.: A61K 49/00, A61K 49/04, A61K 49/18

(54) **Kit and method for perfusion diagnosis**

(71) Applicant: Julius-Maximilians-Universität Würzburg, 97070 Würzburg (DE)
(72) Inventor: Vogel, Benjamin, 97218 Gerbrunn (DE); Wedekind, Georg, 97078 Würzburg (DE)
(74) Representative: Leissler-Gerstl, Gabriele

(57) **Abstract**

A kit for perfusion diagnostic comprises as component (1) microparticles and/or nanoparticles comprising at least one functional group available for binding wherein the functional group is a first reaction partner of an orthogonal system, and as component (2) marker molecules, wherein each marker molecule carries at least one functional group that is a second reaction partner of an orthogonal system.

## Description

The present invention is concerned with a kit and a method for perfusion diagnostic, wherein functionalized nano and/or microparticles are used in combination with functionalized marker molecules.

The diagnostic of perfusion and the determination of perfusion disorders is important with regard to diseases and conditions like cardial infarct, stroke, and other vascular disorders in organs. Perfusion is impeded by an occlusion of a blood vessel which causes ischemic conditions in the area downstream the occluded vessel, the infarction. The occlusion of blood vessels results in insufficient supply of oxygen, toxic metabolites begin to accumulate in cells and tissue is damaged. With an aging population incidence of these diseases is increasing and analytical tools for determining the area of impeded perfusion are necessary.

For studies regarding these diseases animal models are used, where an artificial occlusion is made by ligating a blood vessel using cold coagulation, clipping, a catheter or other techniques. Thereby, perfusion is hindered and the relation between the lack of perfusion and resulting damages at organs, like heart, lung, liver, spine and of tissue are examined. By visualizing the area of infarction and the area of tissue that is still perfused by blood, conclusions can be drawn with regard to the amount and site of the damages. These studies are important and necessary and are made in an early phase of ischemia where it is desirable to differentiate between vital perfused areas and hypoxic non-perfused areas, at a time where it is not possible to observe morphological differences in the tissue.

Presently, two methods are mainly used for visualizing perfusion: the use of dyes that bind to proteins circulating in the vascular system and the use of fluorescent particles which become immobilized because of their size and form in the capillary system and can be detected by their fluorescence.

In perfusion tests, the marking compounds, i.e. dye or fluorescent particle, are injected into an animal after a blood vessel has been artificially occluded and before sacrificing the animal. The substances are distributed in the perfused vascular system and tissues, but not in those parts that are occluded. The dye or fluorescent compound remains in the tissue. Therefore, by using this method infarction areas can be made visible, but the tissue sample remains stained and cannot be used for further diagnostic methods. A model animal that is used for perfusion analytics can be used only for this specific analytical method but it is excluded to conduct further analysis using classical histologic methods, microscopic methods or diagnostic methods, in particular methods that need selective dying. Also the use of methods based on molecular biological analysis or based on protein biochemical assays cannot be carried out or can be carried out only on a limited basis because the dye or fluorescent compound interferes.

A further development in diagnostic methods is the use of fluorescent particles which can be made visible using special filter systems and, which therefore do not interfere with light microscopy and histological stainings. However, state-of-the-art fluorescence-based analytical methods with increasing importance in diagnosis are still largely restricted like fluorescence microscopy, flow cytometry, real-time quality PCR and fluorescence-based Western blots. Due to their strong fluorescence properties interference of fluorescent particles with these methods can never been ruled out completely even with elaborate purification methods.

The above mentioned problems lead to a higher need of test animals and a higher demand for tissue material. This is not desirable with regards to guiding principles and ethics of experimental animal testing (3Rs: reduction, replacement, refinement) economic and scientific reasons.

Summarizing, the methods presently used for perfusion diagnostic allow to use either cheap dyes, like Evans Blue, which allow visibility because of deep penetration due to direct binding to serum proteins and vessel walls, or fluorescent microbeads. However, with Evans Blue staining no determination of different vessel sizes is possible, only one color is used and no filtering out is possible. The perfused tissue is permanently stained and mostly discarded after perfusion analysis due to stated drawbacks. Fluorescent microbeads are useful in depicting different sizes of vessels due to size exclusion by the use of different particle sizes and allow use of embedded different fluorescent colors and surface modifications. Moreover, conventional filter sets can be used to filter out emission. However, the fluorescent microbeads that are used for perfusion purposes have a very strong fluorescence which is not tunable. This makes any subsequent fluorescent analysis difficult or impossible because of the background noise. This mostly excludes the tissue for the use with highly sensitive fluorescence-based diagnostic methods.

A major disadvantage with all these known methods is that tissue samples and often the whole test animal cannot be used in further tests and that tissue samples have to be discarded after the perfusion determination due to predominant fluorescence or stain, and all experiments have to be planned carefully. A change of color or detection method is not possible after perfusion tests. Animal consumption is high and data harvest is low.

Thus, it was the object of the present invention to provide tools and diagnostic methods for the determination of vascular and organ perfusion which allow multiple diagnostic tests, further use of samples and/or animals, high flexibility, the use of different colors, detection methods and order of methods, and at the same time provides reliable results, is efficient and provides for high visibility.

All the drawbacks of the prior art methods are overcome by a kit and a method as defined in the claims.

The kit of the present invention comprises two separate components that are functionalized with reaction partners of an orthogonal system that is not occurring in biology and so cannot interfere with endogenous compounds in the organism: component (1) comprises microparticles and/or nanoparticles comprising at least one functional group available for binding wherein the functional group is a first reaction partner of an orthogonal system, and component (2) comprises marker molecules, wherein each marker molecule carries at least one functional group that is a second reaction partner of an orthogonal system.

The kit is used in a method for perfusion diagnostic on a subject wherein a tissue sample from the subject, that has received an amount of functionalized microparticles and/or nanoparticles for desired distribution of the particle in the organism before taking the sample, is contacted with an amount of functionalized marker molecules under conditions that allow reaction between the first reaction partner of an orthogonal system bound to the microparticles and/or nanoparticles and a second reaction partner bound to the marker molecule; wherein after removal of unbound marker molecules amount and site of marker molecules in the sample can be detected or made visible, respectively. The microparticles and/or nanoparticles have at least one binding partner of an orthogonal system available for reaction with the other partner of an orthogonal system which is bound to a marker molecule. They are administered in due time before taking a sample, i.e. at a time before taking the sample that allows distribution of the particles in the area of interest, for example one minute to 24 hours before the sample is taken or the test animal is sacrificed.

A "subject" can be an animal and comprises any invertebrate or vertebrate animal, including fish, reptiles and amphibians. Usually, the subject is a mammal, such as a human being or a rodent.

The term "orthogonal system" refers to a pair of reaction partners that are either so dissimilar to reaction partners/compounds occurring in nature that they can only interact with them to a very limited extent, if at all, or have reaction partners that are hardly or not available for reaction under normal conditions. In other words, a reaction partner used according to the present invention will not or hardly find a reaction partner in the organism it is applied to either because the reaction partner is not available for reaction or because such type of reaction partner does not occur in the organism. Thus, for example pairs like biotin-avidin/streptavidin, antigen-antibody, or ligand-receptor, cannot be subsumed under an orthogonal system. The orthogonal systems used according to the present invention are built by reaction partners of a "click system", i.e. a component carrying an azide group and a component carrying an alkyne group, or of a pair of complimentary non-natural nucleotide sequences.

The term "non-natural nucleotide " refers to nucleotides comprised of components that are not occurring in nature, such as L-Ribose, and so are resistant to enzymatic decay. Examples for non-natural nucleotides are spiegelmers (L-Ribose), LNA (locked nucleic acids) or PMOs (phosphorodiamidate morpholino oligo). The non-natural nucleotides allow specific base-pairing with a complementary sequence, that can be a complementary nucleic acid consisting of naturally occurring nucleotides, or a complementary spiegelmer, LNA or PMO sequence..

The term "non-natural nucleotide sequence" refers to a nucleic acid sequence that does not occur in the respective organism.

The term "microparticles" as used in the present description shall include particles having a size in the µm range as well as particles having a size in the nm range as well as mixtures of both. Thus, the term microparticles when used to describe the present invention shall include microparticles, nanoparticles and mixtures of microparticles and nanoparticles. The term nanoparticles is used to specifically refer to particles having a size in the nm range. The size of the particles can be determined by sieving, dynamic light scattering, coulter counter or particle imaging.

Microparticles used for the present kit and method can be biodegradable or non-biodegradable. If non-biodegradable microparticles are used, those based on polymers like polystyrene or on silicone polymers, like latex based beads, are useful among others.

The material used for producing the microparticles should be biocompatible, i.e. shall not be detrimental for tissue, cells, organs, or a subject/organism for which it is to be used. Moreover, the microparticles shall not cause undesirable reactions with a sample to be used. Preferably the material used for producing the microparticles is neutral, i.e. does neither react with the subject nor with diagnostic components used in the tests. Materials for microparticles to be used in animals, particularly human beings, for diagnostic and therapeutical purposes are well-known and the skilled artisan can find the most appropriate ones.

The microparticles can be degradable or non-degradable depending on the method they are used in.

In one embodiment the microparticles are made of a biodegradable material. Materials that are biodegradable in physiological environment are well-known in the art. An example are polymers based on polyethylenglycol (PEG), alginate, polylactide and/or polyglycolide (PLG). One advantage of using PLG is that the time period for degradation can be adapted by selecting the rate of lactide and glycolide in the polymer. For microparticles that are used for living animals such as human beings, it is preferred that the microparticles are biodegradable to not be harmful for liver, kidneys or other organs where the microparticles can accumulate. Microparticles that are not biodegradable can cause harm to the vessels or organs where they accumulate or can cause a thrombosis, which is undesirable in living subjects. However, this mechanical vessel obstruction by microparticles can be used by purpose to produce certain effects in live experimental animals if used in an appropriate application scheme.

Thus, in an embodiment of the present invention microparticles are used for administration to a subject to cause an occlusion at a predetermined site to assess the influence of such an occlusion on the environment, for example to assess lung edema.

If the microparticles are used in animal models that are sacrificed after conducting the example, the microparticles can also be non-degradable. Examples for neutral materials that can be used in this case are polystyrene, polyacrylamide, silicium-based compounds, metal based and magnetic compounds.

The microparticles can have different sizes depending on the perfusion method they should be used in. If perfusion in small vessels and capillaries shall be assessed, the microparticles must have a small size, and should in particular be nanoparticles having a size that is big enough to load the microparticles with functional groups but small enough to pass small vessels. On the other hand, if the microparticles are used to assess perfusion in larger vessels or in organs, a higher particle size can be useful. Particle sizes between 1 nm and 100 µm are contemplated depending on the use.

In some embodiments, all microparticles have about the same size, in other embodiments, different lots of sizes are provided, such as a lot of microparticles with a smaller size, for example nanoparticles in the assessment of capillaries, and a lot of microparticles of larger size, such as 100 nm to 10 µm. More than two lots of microparticles with different sizes can be used.

A microparticle used in the kit of the present invention carries at least one group, which is a functional group and is the partner of an orthogonal system. The functional group is bound to the microparticle in a manner that the function, such as an alkyne or azide group, or an artificial nucleotide sequence, is available for binding to the second binding partner. The group when reacted with the functional group that is the second reaction partner results in a strong binding between both partners.

Thus, component (1) can comprise microparticles all having about the same size and all carrying the same type of functional group, or microparticles having different sizes and all carrying the same type of functional group, or two or more lots of microparticles with different size, each lot having one size, and each lot having a different type of functional group, and any other mixture.

The other part, component (2), used for the kit of the present invention is a marker molecule that carries the second reaction partner of the orthogonal system. A marker molecule is a compound that can be detected in a specific manner. In particular, the marker molecule can be any molecule that can be reliably detected, is visible, can be made visible, or is optically detectable.

Detectable molecules can be directly visible, like dyes, can be made visible by excitation, like fluorescent compounds, can be made visible by their radiation, like radioactive isotopes, or can be made visible by a specific reaction, like enzymes. Thus, examples for marker molecules that are useful for the present invention are dyes, fluorescent, luminescent, phosphorescent compounds, compounds comprising radioactive isotopes, or enzymes. As the marker molecule for use in the method of the present invention is used outside the body, toxicity or biocompatibility are not of importance. Since the marker molecule is introduced to the section or tissue material to be analyzed only when the reaction with the other binding partner shall take place, marker molecules cannot interfere with analytical tests performed in the tissue material before. The marker molecule, however, should not destroy the tissue, cell, or organ to be analyzed but preferably should conserve the structure so that the desired perfusion information and also other information can be extracted.

One group of suitable marker molecules are dyes that are used for staining tissue, in particular for staining components in tissue, like membranes, proteins, nucleic core etc. Dyes that are usable for this type of staining are well-known in the art and any known dye can be used as long as it creates a detectable signal and can be derivatized by introducing the reaction partner of an orthogonal system as defined above.

One group of suitable marker molecules are enzymes that are used for indirectly staining or marking tissue by a reaction creating a detectable signal. The sample is specifically stained at the location of the introduced enzyme, e.g. a peroxidase or phosphatase by catalyzing a reaction of components that are also added and/or are present in the surrounding media that causes a signal, for example precipation of a dye, light emission or any other detectable reaction at the location of the introduced enzymes. These reactions are well-known in the art and any known enzyme can be used as long as it can be derivatized by introducing the reaction partner of an orthogonal system as defined above. One example is the combination of nitro-blue tetrazolium chloride (NBT) with diaminobenzidine (DAB) and/or 5-bromo-4-chloro-3'indoylphosphate p-toluidine salt (BCIP), that creates a blue precipitate in the presence of phosphatase. Another example is enhanced chemiluminescence where peroxidase creates a luminescence by reaction with a substrate, such as a luminol-based substrate that produces light in the presence of horseradish peroxidase (HRP).

Another suitable group of marker molecules comprises luminescent, phosphorescent or fluorescent compounds, in other words compounds that emit light in reaction to applied energy, like radiation, chemical reaction, electrical energy. Emission of light is dependent on the applied energy, for example from the wavelength of light. A lot of luminescent, phosphorescent and fluorescent compounds are known in the art. Suitable are in particular those compounds that maintain their luminescent, phosphorescent or fluorescent properties even when carrying a reactive group and when bound, and that emit light that is detectable in the environment where it should be detected, i.e. tissue, organ, or cell.

Examples for fluorescent dyes are ATTO dyes, manufactured and commercially available by ATTO-Tec GmbH, Siegen, Germany, and rhodamine derivatives, commercially available as TAMRA dyes or ALEXA dyes (Molecular probes, Life Technologies).

An important advantage of the present invention is that for detection of perfused tissue areas the marker molecules are added after perfusion of the organism with micro particles. Micro particles alone, distributed everywhere in the organism, are inert and not detectable since they do not contain any dyes or other detectable components. Subsequently the particles itself do not interfere with analytical testing methods post perfusion of microparticles at all. Therefore e.g. in a tissue section proteins, lipids or nucleic acids or other components can be made visible by types of fluorescent, phosphorescent or luminescent compound marker molecules by using methods well known in the art like immunohistochemistry or immunofluorescence without any drawbacks or limitations. If the visualization of perfused areas is desired, this can be made optionally during protocol by adding the marker molecule (2) of the orthogonal system in an appropriate manner. This also allows multispectral approaches as used in e.g. in multi-color fluorescence microscopy as co-localization of perfused areas with other structures in the tissue. Furthermore this approach also allows the use of very bright compounds for the perfusion diagnosis that otherwise would interfere with other diagnostic tests performed with perfused material.

In another embodiment a luminophor can be used as marker molecule which is visible after excitation and glows for some time thereafter.

The marker molecule can also be a compound comprising radioactive isotopes such as ³H, ¹²⁵I, ¹³¹I, ³²P, ⁵⁷Co, ⁷⁵Se, ⁵⁹Fe, ¹⁴C and ³⁵S. In some embodiments it is useful that the marker is very close to the microparticle to be detected. In this case, the marker molecule can be a reaction partner of an orthogonal system which comprises one or more radioactive isotopes. As with the dyes and fluorescent compounds, this is an embodiment which is particularly useful if the sample shall be used to also detect other analytes by radiographic methods, as the labeled conjugate of marker molecule and microparticle of the present invention is created and detected only in a predetermined step, for example the last step of a set of diagnostic tests, and, thus, does not disturb other assays on the same sample or a sample from the same test animal.

The marker molecule comprises the second partner of an orthogonal system. The binding of the second reaction partner to the marker molecule has to be carried out without essentially affecting the marking properties. The functional group providing the second reaction partner can be part of the marker molecule, or can be bound directly or via a linker.

To increase the signal or improve visibility of the marker molecules, respectively, it can be useful to provide the microparticles with a multitude of reaction partners to "attract" more than one marker molecule. Microparticles can be provided either with a multitude of reaction partners or in an alternative linker molecules can be bound to microparticles which carry two or more reaction partners.

An orthogonal system useful for the present invention is built by reaction partners of a so-called "click" system, or by complementary strands of non-natural nucleotides.

In one embodiment of the present invention reaction partners of a click system are used. "Click Chemistry" is a term that is used to describe reactions that are high yielding, broadly applicable, are stereospecific, simple to perform, with a low amount of by-products, in other words thermodynamically-favored reactions that lead specifically to one product. Some reactions of this type are known, such as nucleophilic ring opening reactions of epoxides and aziridines, non-aldol type carbonyl reactions, such as formation of hydrazones and heterocycles, additions to carbon-carbon multiple bonds, such as oxidative formation of epoxides and Michael Additions, and cycloaddition reactions. For the present invention where the reaction partners should not have functional groups that are present in the subject to be analyzed, such as in their tissue or cells, it has been found that in particular the azide-alkyne cycloaddition is useful as reaction for strongly binding reaction partners bound to microparticles and markers. Microparticles as well as marker molecules can be functionalized with azide and alkyne groups. When the conditions allow a cycloaddition reaction then this reaction selectively results in a strong bond in the form of 1,2,3-triazoles.

In the 1960ies Huisgen proposed a thermally activated dipolar 1,3-cycloaddition reaction. This reaction, however, is not preferred as it requires elevated temperatures and often produces mixtures of two regioisomers when using asymmetric alkynes. Therefore, this classic 1,3-dipolar cycloaddition is not subsumed under a true click reaction. Instead, it is suitable to use a catalyzed variant. Some catalysts are known to catalyze the cycloaddition. The best known reaction is a copper-catalyzed version that allows the synthesis of 1,4-disubstituted regioisomers specifically. Another variant is a ruthenium-catalyzed reaction that provides for an opposite regioselectivity with the formation of 1,5-disubstituted triazoles. Both variants can be used for the present invention.

The copper-catalyzed reaction has been further developed to avoid side reactions that are caused by using Cu(I) as catalyst. It is known that in the copper catalyzed reaction reactive oxygen species are generated which not only cause toxicity issues for living cells but can also disturb experiments with tissue samples, for example by induction of structural damage of the cytoskeleton. Moreover, fluorescence can be extinguished or shifted. To overcome these disadvantages, an enzymatic or chemical oxygen scavenger system can be used. Therefore, in one embodiment of the present invention, the kit comprises in addition to components (1) and (2) an oxygen scavenging system. Such a system can also be used in the method of the present invention. Preferably, a so-called ClickOx protocol which has been developed to reduce ROS-associated damage, is applied. The ClickOx system relies on an enzymatic system comprising glucose oxidase, ß-D-glucose and catalase.

Thus, in one embodiment of the present invention click chemistry based on a azide-alkyne cycloaddition is used as a prototype click reaction, optionally together with an oxygen scavenging system.

When using click chemistry, the functional groups that react with each other are bound to components (1) and (2) of the present kit. The number of functional groups bound to a component can vary and depends on the structure and type of the component, the surface thereof, the reactivity etc. Each microparticle of component (1) and each marker molecule of component (2) should carry at least one functional group. The number can be higher for a microparticle and smaller for a nanoparticle and small for a marker molecule. Thus, a marker molecule might carry only one, two or three functional groups, whereas a microparticle can be loaded with a higher number depending on particle size, density of functional groups on or within the particle with functional groups. In one embodiment of the present invention, all microparticles of component (1) carry the same type of functional group and/or all marker molecules of component (2) carry the same type of functional group. In another embodiment microparticles of component (1) are loaded with different functional groups and/or marker molecules of component (2) carry different functional groups. In the latter embodiment, microparticles that are loaded with different functional groups can differ in other regards, such as size or type, or can be the same. Moreover, in the latter embodiment component (2) can comprise different marker molecules loaded with different types of functional groups.

There are several options for combining components and functional groups which then allow for many variants of diagnostic methods.

In one embodiment one of the components - microparticles or marker molecules - carries azide groups and the other one of the components carries alkyne groups. Under conditions that allow the reaction of both groups, they will react and by this reaction marker molecules are bond to microparticles.

Providing microparticles and marker molecules with at least one, suitably more than one, such as a plurality of azide groups or alkyne groups can be done with standard chemical reactions that are known to the skilledperson. Thus, it is well-known in the art to functionalize microparticles via groups that are present at the polymer that forms the microparticle. The polymer forming the microparticle can also carry linkers that provide sites for coupling one or more functional groups.

The groups should be bound such that they are available for reaction. Functional groups that are bound directly, such as at the surface of a microparticle, provide for a tight bonding, i.e. a small distance between microparticle and marker. Linkers can provide for a predetermined distance between marker molecule and microparticle and can provide for availability of the group. Furthermore, linkers can provide more than one binding site to bind more than one marker molecule at one site.

Alternatively, azide and/or alkyne groups can be generated directly by a chemical reaction.

Marker molecules can be functionalized via binding groups that are present in the molecule or via an added linker. The functional group can be bound directly or via a linker.

If a linker is used, it can be a bridge having up to 50 atoms selected from C, O, S, P, in particular up to 20 atoms. In general there is no upper limit for the length of the linker except that it should have a size that the functional groups are in a suitable place for reaction.

Microparticles can carry more than one type of functional group to allow binding of more than one marker molecule. This can be useful for detection strategies to allow more sophisticated search strategies without the need for more animals. For example, component (1) can comprise different lots of microparticles, for example having different size, and all microparticles carry the same type of a first functional group and each lot carries a different further type of functional group. This can be useful when perfusion in the live animal has to be determined time resolved e.g. before and after an artery occlusion in the heart. This is performed by injection of one lot of particles before artery occlusion (everything perfused) and an injection of another lot of particles after occlusion (occluded areas not perfused). The different microparticles in this approach are equipped with different functional groups for the individual detection of the microparticles e.g. first particles: azide groups, second particles: alkyne groups. Different vessel sizes can also be excluded in this approach by choosing different microparticle sizes.

Different functional groups can be protected in known manner, such as described in "Protective Groups in Organic Synthesis" von Theodra W. Green, 1981, John, Wiley & Sons, Inc., and can be activated when needed. For example, microparticles can carry azide groups as well as alkyne groups. The presence of both groups can result in crosslinking of microparticles when the conditions for the bipolar cyclo addition are applied. This can be desirable for some strategies. If crosslinking shall be limited, one type of reaction partner can be protected and can be activated only when the first reaction partner has reacted with functional groups on the or one of the marker molecules.

In another embodiment of the present invention the partners of an orthogonal system are non-natural nucleotide sequences, i.e. sequences comprising at least one nucleotide not found in nature. Such sequences can be obtained as is known to the skilled person, in particular by using at least one type of nucleotides that is not found in nature. Examples are nucleotides comprising non-natural sugar moieties, base derivatives not found in natural nucleotides, etc. Useful as partners of an orthogonal system are nucleotide sequences comprising morpholinos, spiegelmers, LNAs, or mixtures of those that allow, not necessary complete, complementary base pairing. Such artificial nucleotides are not degraded by exonucleases or endonucleases that are present in the organism.The oligonucleotide strands can have a length such that a strong bond after reaction is present. The length can be in the range of up to about 30 nucleotides, although this upper limit is not critical. The strands should have at least 5 nucleotides to allow strong binding. Hybrid base pairing such as known from single-stranded DNA and RNA are also conceivable for certain natural and non-natural nucleotide sequence combinations to achieve an orthogonal system.

For detection of microparticles carrying non-natural nucleotide sequences, marker molecules carrying at least one complimentary sequence are used. The nature of the complimentary sequence is uncritical as it is used outside the organism. It can be a sequence of spiegelmers, LNAs, morpholines etc. or a sequence of naturally occurring nucleic acids (DNA or RNA). In one embodiment the sample that is used for detection can be deprived from DNA and RNA of the organism by digestion with enzymes like exonucleases or endonuclease, as those enzymes cannot digest the non-natural sequences bound to the microparticles.

The sequence used is also uncritical as long as it bonds with its compliment in sufficient strength. Thus, the sequence can be homologous or heterologous for the organism. In one embodiment sequences that are heterologous for the organism to be examined, are used.

The kit of the present invention is very versatile because the microparticles as well as the marker molecules can be adapted to a specific assay by adapting the size of the microparticles, the loading of the microparticles, the selection of reaction partners, the amount of binding sites, the number and type of marker molecules.

The kit of the present invention can be used in a method for perfusion diagnostic.

Thus, a further aspect of the present invention relates to a method for perfusion diagnostic that comprises the steps:
a) providing a sample of a tissue of a subject that has received an amount of functionalized microparticles and/or nanoparticles for a time period to allow proper distribution of the particles before taking the sample;
b) contacting the sample or a part or section of the sample with an amount of functionalized marker molecules under conditions that allow reaction between the first reaction partner of an orthogonal system bound to the microparticles and/or nanoparticles and a second reaction partner bound to the marker molecules;
c) removing unbound marker molecules; and
d) analyzing amount and site of marker molecules in the sample.

A sample or section of a tissue, organ or cell of a subject can be obtained as is known to the skilled artisan. The sample can be taken from a living animal, such as a living mammal or after sacrificing the subject. Before the sample is taken, an amount of functionalized microparticles and/or nanoparticles as defined before has to be administered to the subject such that the microparticles are delivered to the site that is to be inspected. Administration can occur as known, such as enterally or parenterally, and either systemically or locally, via means like an infusion, an injection, a solution, etc.

It has been found that for analysis sections having a thickness of about 5 to about 40 µm are suitable. Sections with this thickness can withstand intense washing and chemicals used for the click reaction. It has been found that thicker sections, such as 20-30 µm sections, are particularly useful for visualization for the eye. For this approach usually frozen sections are used. The reason is that frozen tissue material is still fully available for other subsequent analysis after sectioning since it is not fixed by fixatives as formaldehyde. So the remaining material can be divided by interpolation of the perfusion information of the section to the remaining tissue material into perfused and not perfused areas for subsequent analysis.

After a time period that is sufficient to allow the microparticles to be distributed in the area to be analyzed, a sample can be obtained. In case of a living body, the sample can be obtained by biopsy. In case of an experimental animal an organ extraction can be performed.

For diagnosis a sample/section is contacted with an amount of functionalized marker molecules under conditions that allow reaction between the functional groups on the microparticles as first reaction partner and the functional groups of the marker molecules as second reaction partners. The conditions to be applied depend from the specific binding partners and are commonly known for the applied reactions.

After reaction of the functional groups, non-reacted components are removed, usually by one or more washing steps. Thereafter the amount and site of the reacted binding pairs can be analyzed by detection, optionally amplification and visualization of the signal of the marker molecule. The signal can be optical, electronical, or radioactive and can be detected, optionally amplified and made visible by known means.

In a preferred embodiment, when the orthogonal system is a click system, an oxygen scavenging composition is added to the sample to avoid harmful side-reactions in the tissue as well as in the components. A particularly useful oxygen scavenging composition comprises enzymes that destroy active oxygen species. It has been found that using a composition comprising glucose oxidase, ß-D-glucose and catalase conserves tissue very efficiently and thus contributes to a reliable diagnostic system.

The method of the present invention allows to analyze perfusion in organs like heart, liver, kidney, spleen, and provides a tool for control and analysis of perfusion in vessels, tissues and organs. As the size of the microparticles can be adapted, the method can be used to visualize perfusion of big vessels as well as of small capillaries by size exclusion.

The present invention provides a method for perfusion diagnostic that allows to analyze perfusion in a human being as well as in test animals. When using the method of the present invention the samples can be used for further tests without interference or drawbacks to the tissue material and can optionally provide information about perfusion in different types of organs and vessels even long after tissue harvesting and even if such analysis was originally not intended. The detection of the perfused inert microparticles can be performed by any method, known or still unknown, as long as it can be targeted specifically to the microparticle by the orthogonal system. This was not available before or was not available in one experiment before. This allows a more flexible usage of experimental animal tissue, which follows the worldwide guidelines of the 3R's (replacement, reduction, refinement) for experimental animal research and is a clear example for reduction and refinement.

Furthermore, the present invention allows to visualize occluded vessels, permeable vessels and perfused areas. To make a differential diagnosis, microparticles of different sizes can be injected into an animal for example after an event like a myocardial infarction. Affected tissue sections can then be contacted with different dyes which bind to the different types of microparticles and allow to show the occlusion site, perfused vessels and bleeding areas.

The Figures and the examples that follow shall further explain the gist of the present invention without limiting it.

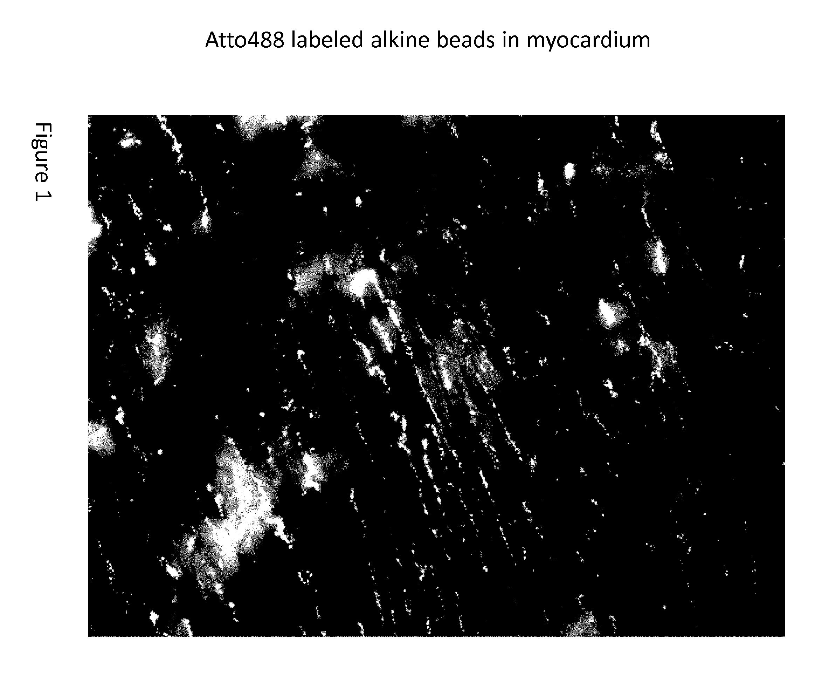

In the Figures
Fig. 1 shows a frozen section of ATTO 488 labelled alkyne beads in perfused mouse myocardium.

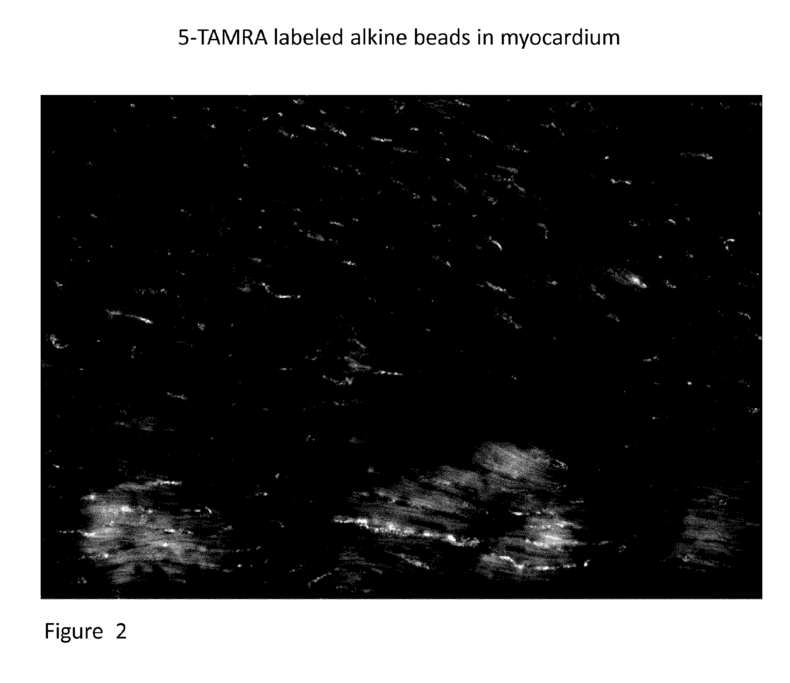

Fig. 2 shows a frozen section of 5-TAMRA labelled alkyne beads in perfused mouse myocardium.

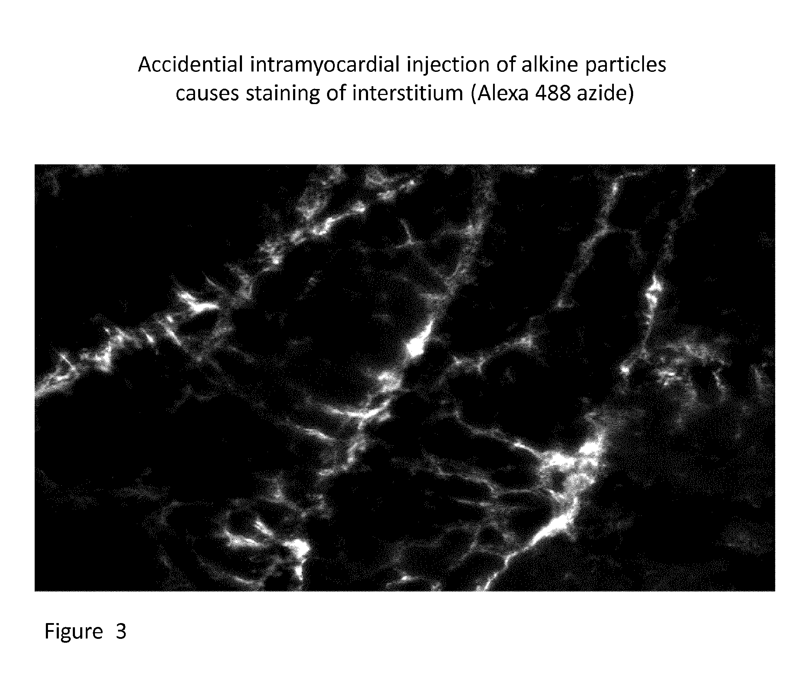

Fig. 3 shows the distribution of alkyne beads labelled by ATTO 488 azide in a frozen section. In this experiment microbeads were injected via the apex of the myocardium instead via the leg vein. Damage to the heart tissue at the injection site caused bleeding into the interstitial space which could be visualized after addition of ATTO488 by dye labelled alkyne microbeads.

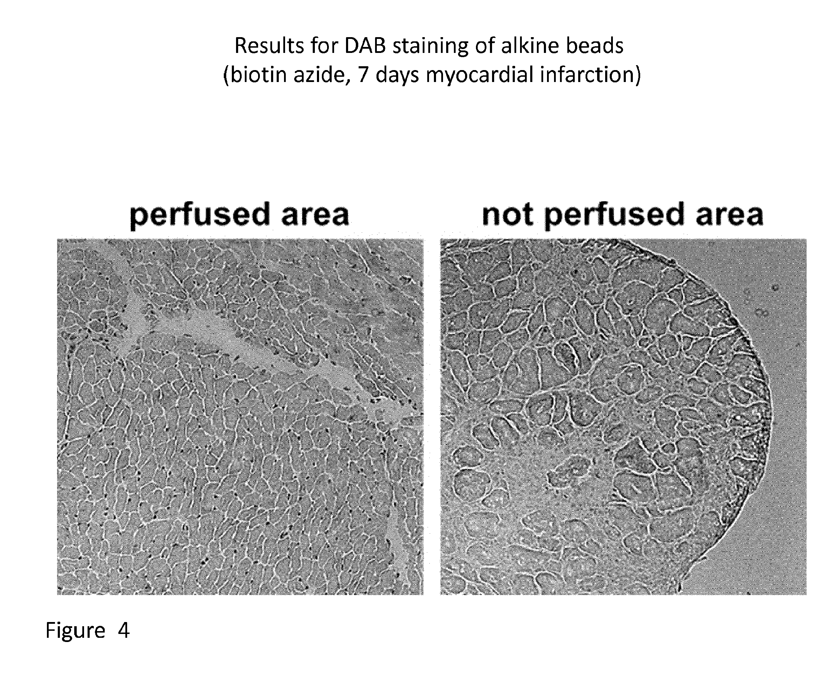

Fig. 4 shows a frozen section of infarcted myocardium (7 days after induction of myocardial infarction) of a mouse perfused with alkyne microbeads visualized by DAB staining (marker molecule was biotin azide, biotin was detected by a streptavidin-horse radish peroxidase, peroxidase is used for DAB reaction), wherein the left panel shows the perfused area, whereas the right panel shows the not perfused area. Vessels in perfused areas are stained in dark brown. No stained vessels can be found in not perfused area.

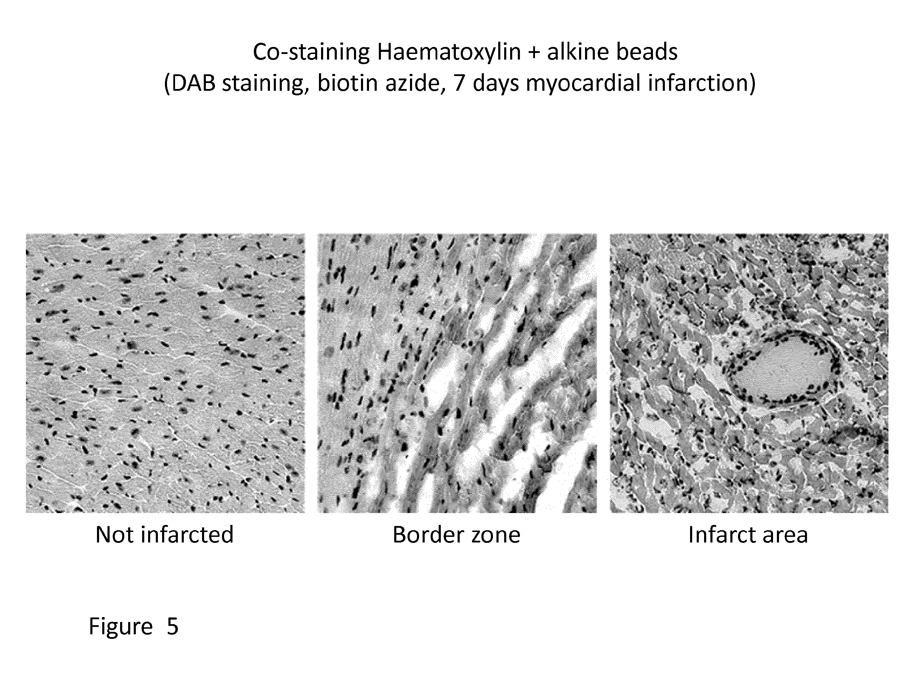

Fig. 5 shows a frozen section of myocardium stained with haematoxylin for nuclei and DAB for perfused vessels in a multi-color approach. Cell nuclei have been stained with haematoxylin and are stained faintly blue. Perfused vessels are visualized by using DAB staining. Alkyne microbeads in vessels stained dark brown (left panel). The medium panel shows the border zone of an infarcted myocardium characterized by the occurence of not infarcted (left side of medium panel) and infarcted tissue (right side of medium panel). In the infarct zone staining of cell nuclei is abolished due to cell necrosis and nuclei rupture. A general diffuse brownish staining can be observed in the infarct zone. This indicates bleeding into the infarct zone. Dark brown shapes in the infarct zone indicate that there is anothermicrobeads accumulation. A probable explanation for this is that infiltrating cells as macrophages take up microbeads bleeding from the bloodstream into the infarct zone. The right panel shows non infarcted part of the myocardial tissue with a large vessel. Within this large vessel blood cells attached to the vessel wall can be detected. These cells are stained brownish indicating that microparticles were engulfed by these cells by phagocytosis directly from the blood stream. This underlines that microparticles can also be engulfed by phagocytosing cells.

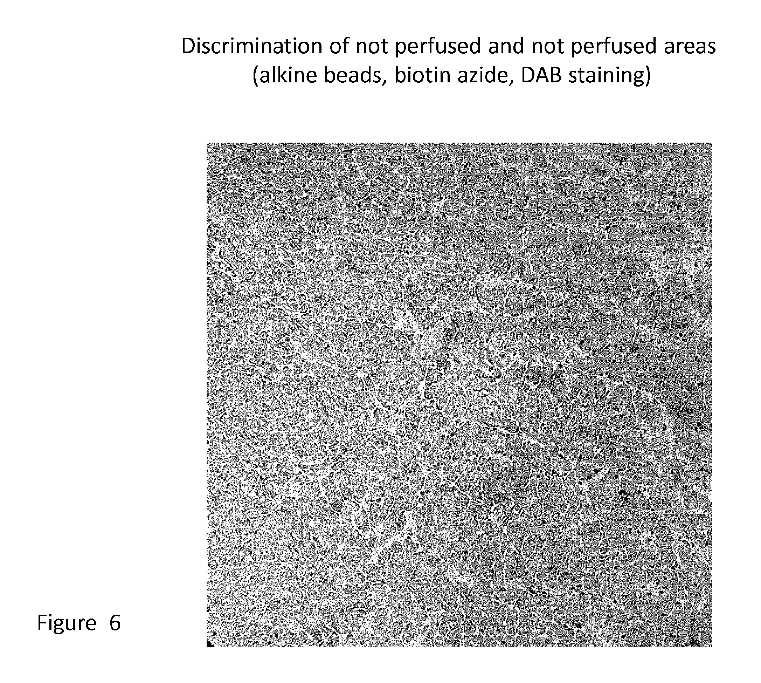

Fig. 6 shows a frozen tissue section of an infarcted myocardium 30 minutes after experimental induction of myocardial infarction in mice. Within this field of view perfused (right side) and not perfused (left side) areas are clearly discriminable by distinct DAB staining of alkyne microbeads located in vessels surrounding cardiomyocytes. Note that non obvious tissue damage occurs after 30 minutes of ischemia, only the perfusion changes distinctly within the tissue locally due to the induction of myocardial infarction.

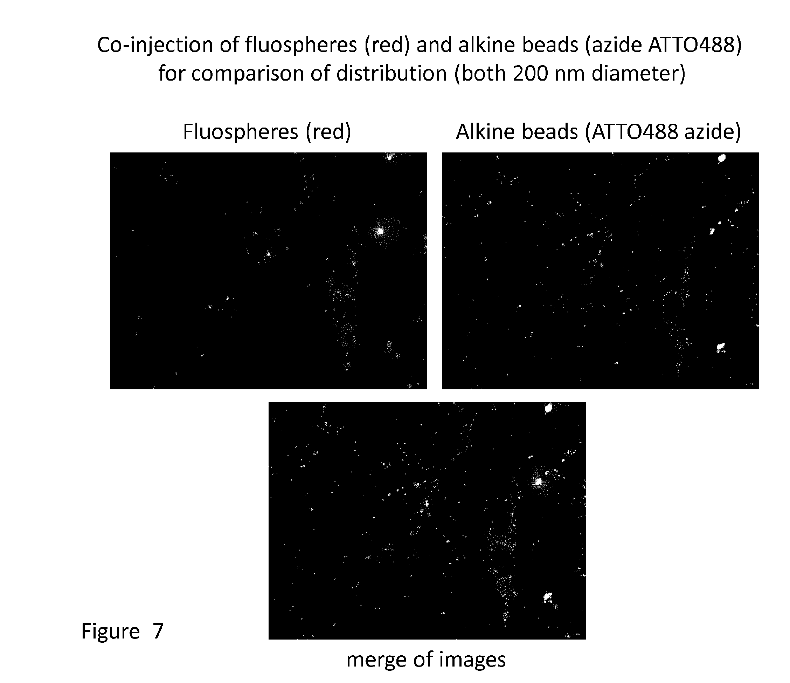

Fig. 7 shows frozen tissue sections of myocardium wherein different types of microparticles, alkyne beads and microbeads with a permanent red fluorescence (Fluospheres) have been injected in 1:1 ratio, wherein all microparticles had the same size (200 nm diameter). The left panel shows alkyne microparticles according to the present invention labelled with ATTO 488azide. The right panel shows the same field of view but shows microbeads with permanent red fluorescence (Fluospheres). The lower panel shows a merged image of both types of visualized microparticles. Alkyne microbeads labelled with ATTO 488 azide demonstrated a much more detailed distribution in the vessels than conventional permanent red fluorescent microbeads (Fluospheres).

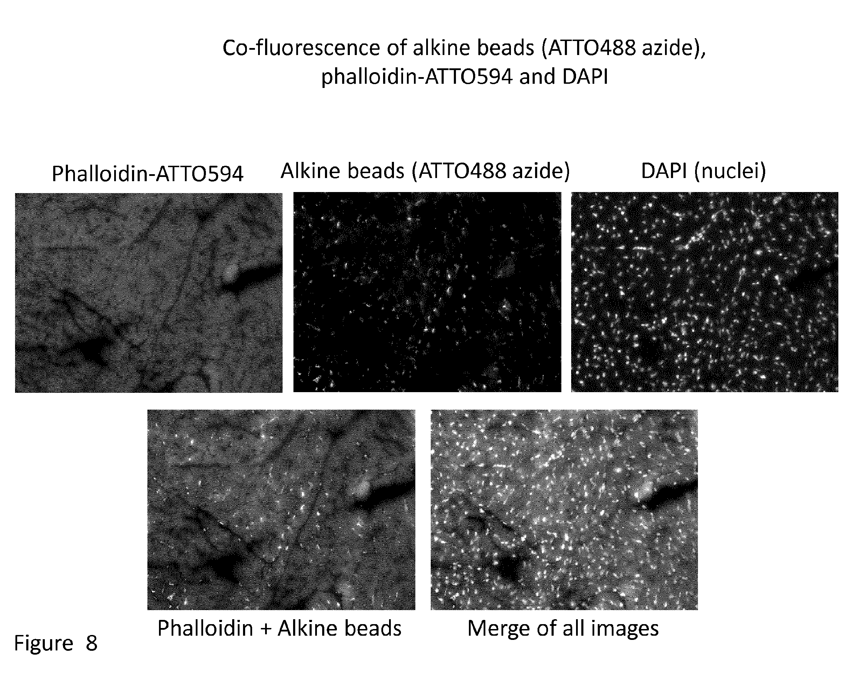

Fig. 8 shows the visualization of alkyne microparticles of the present invention in a multicolor approach with a total of 3 different colors. In the upper left panel nuclei were stained with DAPI. In the upper middle panel microparticles carrying alkyne groups had been contacted with ATTO 488 dye carrying an azide group. In the upper right cardiomyocytes were stained with phalloidin-ATTO 594. In the lower left panel a merge of phalloidin ATTO 594 and alkyne microparticles labelled with ATTO 488 azide is demonstrated. In the lower right panel a merge of all used channels is presented.

### Examples

### General:

The kit of the present invention has been used for perfusion tests using fluorescence and light microscopy. Polystyrene beads having a size of 200 nm were provided with alkyne groups (in the following "alkyne beads"). Furthermore, ATTO 488, 5-TAMRA fluorescent dyes or a biotin moiety carrying an azide group were used as marker molecules for direct detection or to provide a linker for enzyme attachment via streptavidin for DAB staining.

Alkyne beads according to the present invention were injected into mice via the leg veins some days after a myocardial infarction or without myocardial infarction. The beads were distributed (confirmed) within the whole animal within one minute after injection and detectable in all vessels and organs. The mouse was then sacrificed, organs harvested and snap frozen gently embedded in Tissue-Tek with -20°C 2-methyl-butane on dry-ice for later analysis and superior tissue conservation. Sections from frozen tissues were made and ATTO 488 or 5-TAMRA dye or biotin functionalized with azide groups were added to the tissue section. The azide group of the dye reacted with an alkyne group of the bead and the dye or biotin became covalently bound to the bead.

### Example 1

As can be seen in Fig. 1, the alkyne beads are clearly visible and are found in the vessels in the heart. The figure shows transversal sections of the vessels in the heart. The beads functionalized with alkyne groups and no attached marker molecules have no fluorescence.

Another section of the frozen tissue was reacted with 5-TAMRA dye that had been functionalized with an azide group. As can be seen in Fig. 2, the beads are clearly visible within the vertically cut vessel.

### Example 2

The kit of the present invention was used for visualization of intracardial bleeding. Polystyrene microparticles having a size of 200 nm and functionalized with alkyne groups were injected into a mouse via the cardiac apex. This causes tissue damage and bleeding. Sections of frozen tissue from the heart were made for analysis. The sections were reacted with a dye carrying an azide group, the dye used was Atto 488. The azide group of the dye reacted with the alkyne group of the microparticles and thereby became covalently attached. As can be seen in Fig. 3, microparticles are found not only in the vessels but also in the interstitial space due to bleeding from the cardiac lumen. The fluorescence in the interstitial space is clearly visible due to microparticle accumulation. This effect can be used for analysis. E.g. after a cardiac infarction cell necrosis occurs and by cell necrosis the barrier between vessel system and interstitial space is destroyed causing tissue in bleeding. This tissue bleeding can be made visible by the method of the present invention.

Thus, the kit of the present invention allows visualizing and differentiating perfused vessels, occluded vessels (no perfusion) and permeable vessels or tissue (bleeding into tissue) optionally without causing interference to other methods performed with the tissue. To make a differential diagnosis, microparticles of different sizes (exclusion of certain vessel sizes) or with different functional groups of the orthogonal system for multistaining of different microparticles at the same time can be injected into an animal for example before and after an experimentally induced event like a myocardial infarction. Tissue sections can then be contacted with different dyes with different functional groups of the orthogonal system, which bind to the different types of microparticles specifically. This allows to show the occluded, not perfused infarct region, perfused vessels and tissue and bleeding into tissue or different localizations in a time resolved manner e.g. before and after vessel occlusion.

### Example 3

The kit of the present invention was used for immunohistological tests using light microscopy. Sections were used for light microscopy and visualization of microbeads by DAB staining. The tissue used was from an infarcted heart after 7 days or 30 minutes. Alkyne beads according to the present invention were injected. Sections of the frozen heart tissue were made and reacted with a biotin compound functionalized with an azide as marker molecule for the orthogonal system. Before adding the biotin compound all endogenous biotin and avidin molecules were saturated by a commercially available kit (Avidin/Biotin Blocking Kit (SP-2001), Vector Labs). The azide groups of the biotin compound specifically reacted with azide groups of the microparticles resulting in covalently bound biotin to the microparticles. Not bound biotin compounds were washed away. The biotin moiety is readily detected by a streptavidin-peroxidase compound which produces a brownish DAB precipitate at the site of the microbead only. Endogenous peroxidases were saturated by an initial blocking step.

As can be seen in Fig. 4 (left panel), the perfused area which was not affected by the infarction is clearly visible in light microscopy by DAB staining which is localized in the vessels only. The not perfused area (right panel) visualizes that part of the heart where the infarction occurred. No DAB precipitate is visible, so no microbeads reached this part of the heart due to occluded vessels, indicating no perfusion. This is also demonstrated in one image in Fig. 6

In this example, a peroxidase was used as marker molecule. This enzyme was attached to the alkyne microbeads briefly by first blocking all endogenous biotin, second attachment of biotin bound to an azide, third attachment of a streptavidin molecule covalently bound to a peroxidase to the biotin, for a DAB reaction which causes a brown DAB precipitate at the location of the peroxidase at the microbeads located in vessels.

### Example 4

In this example a two-color histological staining in light microscopy was tested, i.e. one staining with a haematoxylin dye to stain cell nuclei, and another staining with the kit of the present invention for visualizing perfusion by DAB as explained.

Sections from frozen tissue were used which had been obtained as described in Example 3.

For diagnosis and confirmation purposes, both cell nuclei and perfused vessels were stained in the myocardium. Therefore, in a first step, the section was contacted with a commercial DAB reagent (Abcam). The addition of the DAB reagent causes DAB to precipitate specifically at the location of the peroxidase enzyme streptavidin conjugate which is attached via the biotin moiety of the biotin-azide compound covalently bound to the alkyne beads via the orthogonal system in a click reaction. The DAB precipitation reaction was observed using a microscope and stopped after sufficient staining by washing with water. In a second step, staining of the cell nuclei was obtained by using haematoxylin after sufficient DAB staining which stains the nuclei which then can be diagnosed by their faintly blue color. The haematoxylin staining was performed only for 1 minute after DAB staining to prevent too strong staining as known by the skilled artisan right before dehydration step. The section was then dehydrated in a rising ethanol row, than xylene and rotihistol (Roth) known to the skilled artisan. Section was finally embedded in Entellan.

### Example 5

In this example a comparison has been made between conventionally used microparticles (Fluospheres, red, F8810, Life Technologies) and a kit of the present invention in a perfusion test to show that alkyne microbeads behave like conventional fluorescent microbeads.

A mouse was injected with a 1:1 mixture of conventionally used microbeads, i.e. microparticles having a size of 200 nm carrying a red fluorescing dye (Fluospheres, red, Life Technologies, Molecular Probes, F8810) and microparticles of the present invention having a size of 200 nm carrying alkyne groups. The beads were distributed within the mouse within one minute. The mouse was sacrificed, the heart was removed and snap-frozen. Sections of the frozen cardiac tissue were made and contacted with ATTO 488 dye carrying an azide group. The alkyne groups of the ATTO 488 dye reacted with the alkyne groups of the beads. Unbound dye was removed in a washing step.

As can be seen in Fig. 7, the conventional beads are found only in larger vessels, whereas the microparticles used according to the present invention are found in small as well as larger vessels with a much higher detail but less intensive. Although the conventional microbeads have a higher fluorescence, the diagnostic value is not as high as with the kit of the present invention demonstrating much higher perfusion details. Single and merged channels are presented.

### Example 6

Multicolor fluorescence approaches with alkyne beads

Sections from frozen cardiac tissue were prepared as described in Examples 1 and 2. For visualization of perfusion ATTO 488 azide was used. Perfusion within transversal sections of the myocardium are shown with green color in Fig. 8, whereas the myocardium itself (visualized by phalloidin-ATTO 594 staining) is shown in red color. Cell nuclei have been stained with DAPI and are shown in white. The images in the lower panels shown in Fig. 8 are merged images produced from the three different fluorescence channels.

The present invention allows the use of other fluorescent marker molecules for visualization of perfusion than ATTO 488. 5-TAMRA and Alexa 647 azide also have been successfully applied in this approach. In principle the present invention allows to choose freely from dyes of all kinds of fluorescence spectra commonly used by the skilled artisan in fluorescence microscopy. This allows visualization of perfusion together with other targets (e.g. antibodies or lectins) for multi-colour approaches for fluorescence microscopy without any spectral limitation or potential background staining.

Frozen sections were stained using a kit of the present invention.

Mice were injected with polystyrene microparticles having a size of 200 nm and carrying alkyne groups, referred to as alkyne beads in the following. If not stated otherwise, for perfusion determination the isoflurane anesthetized mice were injected with 400 µl [1% (v/v) alkyne beads (or 0.5% alkyne beads + 0.5% Fluospheres, red) in 400 µl 1% BSA in 0.9% NaCl] via the leg vein. Beads were allowed to be distributed in the organism for 1 minute by the circulatory system before sacrifice and tissue harvest as described above. To visualize perfusion the following staining protocol was used.

### Frozen section preparation

- Tissue was embedded in TissueTek and gently frozen on dry ice using ice-cold 2-methyl butane.
- Frozen sections were prepared with 10 and 20 µm thickness
- 10 µm sections were used for fluorescence tests, 20 µm sections were used for histological and DAB staining
- Frozen sections for analysis should be prepared fresh and stored in -20 °C after cutting.

### Fixation

- For fixation of frozen sections ice-cold methanol was used for 10 min, then ice-cold acetone for 3 min, then sections were air dried for 10 min.
- Only for sections to be used for histological and DAB staining the following steps were performed:
   ∘ Sections were placed in methanol with 0.3% H₂O₂ for 10-30 minutes to quench endogenous peroxidases
   ∘ Sections were rinsed in methanol to remove residual peroxide.
   ∘ Sections were blocked with avidin (1 drop in 250 µl PBS) 10 min, 50 µl per section (Avidin/Biotin Blocking Kit (SP-2001), Vector Labs)
   ∘ Sections were washed with PBS for 5 minutes
   ∘ Sections were blocked with biotin (1 drop in 250 µl PBS) 10 min, 50 µl per section (Avidin/Biotin Blocking Kit (SP-2001), Vector Labs)
- Sections (all sections: fluorescence + histology and DAB) were washed with PBS for 5 minutes

### Click-protocol

- Sections were rinsed in PBS to rehydrate and to prepare for Click-reaction
- For detection of alkyne beads by immunofluorescence ATTO 488-azide, 5-TAMRA-azide or Alexa 647-azide was used. It has been found that the present invention works perfectly with ATTO 488, 5-TAMRA and Alexa 647 dyes to determine perfusion in different tissues. However the best signal to noise ratio of all tested dyes was observed with Alexa 647.
- For histological staining of alkyne beads by DAB precipitation a biotin-azide was used as marker molecule in the orthogonal system (BCFA-003, BaseClick GmbH). Biotin moiety was detected by a peroxidase-streptavidin compound
- A Click reaction without the combination of 10% Glucose /10% Glycerin and Catalase / Glucose Oxidase component can also be used. However as the protocol without ClickOx components is less efficient that with ClickOx, the conventional Click reaction should be repeated 3 times to reach sufficient signal and staining.
- For immunofluorescence (azide-dyes) one ClickOx reaction is sufficient for staining the alkyne beads in the section. For histologic and DAB staining two ClickOx reactions were found to be sufficient.
- Between all repeated click reactions short PBS washing steps (3 minutes) should be performed.
- ClickOx or conventional Click reactions (details in the following table) were added for 10 minutes each.

Catalase / Glucose Oxidase Mix is:
25mM KCI, 22mM Tris/HCl (pH 7,4), 4mM TCEP (used optionally for storage), 2000U/mL glucose oxidase (Sigma: G2133-50KU), 40000U/mL catalase (Sigma: C1000), and 50% glycerol (adapted from Löschberger et al, 2014)
Table for ClickOx protocol (substances in columns 5 and 6 are left out for conventional ClickOx protocol; amounts are in µl; # number of sections to be stained):

| **1** | **2** | **3** | **4** | **5** | **6** | **7** | **8** |
|---|---|---|---|---|---|---|---|
| **Final conc.** | | 100mM Tris | 5mM CuSO₄ | 1% Glucose/ 1% Glycerin | 1:50 | 4µM Azide | 100mM ascorbic acid |
| # | **H₂O** | **2M Tris** | **100mM CuSO₄** | **10% Glucose /10% Glycerin** | **Catalase / Glucose Oxidase** | **1mM azide** | **0,5M ascorbic acid** |
| **1** | 77 | 6,25 | 6,25 | 12,5 | 2,5 | 0,5 | 25 |
| **2** | 154 | 12,5 | 12,5 | 25 | 5 | 1 | 50 |
| **3** | 231 | 18,75 | 18,75 | 37,5 | 7,5 | 1,5 | 75 |
| **4** | 308 | 25 | 25 | 50 | 10 | 2 | 100 |
| **5** | 385 | 31,25 | 31,25 | 62,5 | 12,5 | 2,5 | 125 |
| **6** | 462 | 37,5 | 37,5 | 75 | 15 | 3 | 150 |
| **7** | 539 | 43,75 | 43,75 | 87,5 | 17,5 | 3,5 | 175 |
| **8** | 616 | 50 | 50 | 100 | 20 | 4 | 200 |
| **9** | 693 | 56,25 | 56,25 | 112,5 | 22,5 | 4,5 | 225 |
| **10** | 770 | 62,5 | 62,5 | 125 | 25 | 5 | 250 |

Sections were rinsed in 10 mM EDTA in PBS for 5 minutes three times after completion of all ClickOx reactions

### Post-staining immunofluorescence (dye-azide)

- After ClickOx reaction protocol as described above, any antibody or lectin or other staining can be applied afterwards
- Co-staining with phalloidin and DAPI is recommended for correct localization of beads in the context of tissue for perfusion determination.
- Wash in PBS and mount in Mowiol as known by the skilled artisan.

### Post-biotin processing for DAB stain

The following protocol can be used for staining in case biotin azide has been used as marker binding component:
- Add ultra-sensitive Streptavidin- HRP polymer in PBS (1:200, Sigma, S2438) and incubate for 30 minutes, RT
- Wash sections twice in PBS for 5 minutes
- Perform DAB staining using Abcam DAB staining Kit
- 500 µl DAB Buffer + 10 µl DAB + 25 µl DAB Enhancer, mix thoroughly and add to sections
- Observe DAB staining for 3-5 minutes until desired staining is reached.
- Rinse sections in distilled water
- Place sections in 50% Ethanol for 1 min
- Place sections in 70% Ethanol for 1 min
- Place sections in 96% Ethanol for 1 min
- Place sections in Ethanol/Xylene for 5 min
- Place sections in Xylene for 5 min
- Place sections in Rotihistol for 5 min
- Place sections in Rotihistol for 5 min
- Mount sections in Entellan

## Claims

1. Kit for perfusion diagnostic comprising
a) as component (1) microparticles and/or nanoparticles comprising at least one functional group available for binding wherein the functional group is a first reaction partner of an orthogonal system,
b) as component (2) marker molecules, wherein each marker molecule carries at least one functional group that is a second reaction partner of an orthogonal system.

2. Kit according to claim 1, wherein first and/or second reaction partner groups are bound directly or via a linker.

3. Kit according to claim 1 or claim 2, wherein component (1) comprises microparticles and/or nanoparticles of different size, microparticles and/or nanoparticles carrying functional groups of different type, and/or a mixture thereof.

4. Kit according to one of the preceding claims, wherein component (2) comprises at least one marker molecule carrying one type of functional group, or a mixture of marker molecules carrying different types of functional groups.

5. Kit according to one of the preceding claims, wherein the marker molecule is a dye, a luminescent, fluorescent, or phosphorescent compound, a compound comprising at least one radioactive isotope, or an enzyme.

6. Kit according to one of the preceding claims, wherein one of the reaction partners is an azide group and the other one is an alkyne group.

7. Kit according to one of claims 1 to 5, wherein the reaction partners of an orthogonal system are complementary oligonucleotides comprising non-natural nucleotides.

8. Kit according to claim 7, wherein the oligonucleotides comprise morpholinos, locked nucleic acids, or nucleotides comprising L-sugar groups, or a mixture thereof.

9. Kit according to one of claims 2 to 8, wherein the linker is a bridge having a length of up to 20 atoms selected from C, O, S, P.

10. Kit according to one of the preceding claims, wherein the kit additionally comprises an oxygen scavenging composition, wherein optionallz the oxygen scavenging composition comprises glucose oxidase, ß-D-glucose and catalase.

11. Method for perfusion diagnostic comprising the steps:
a) providing a sample of a tissue of a subject, that has received an amount of microparticles and/or nanoparticles, functionalized with at least one first reaction partner of an orthogonal system, before taking the sample;
b) contacting the sample with an amount of marker molecules, each marker molecule carrying at least one second reaction partner of an orthogonal system, under conditions that allow reaction between a first reaction partner of an orthogonal system bound to a microparticle or nanoparticle and a second reaction partner bound to a marker molecule;
c) removing unbound marker molecules; and
d) analyzing amount and location of marker molecules in the sample.

12. Method of claim 11, wherein the microparticles and/or nanoparticles are as defined in one of claims 1 to 9 and/or wherein the marker molecules are as defined in one of claims 1 to 9.

13. Method of one of claims 11 or 12, wherein microparticles are used, all having about the same size and all carrying the same type of functional group, or microparticles having different sizes and all carrying the same type of functional group, or two or more lots of microparticles with different size, each lot having one size, and/or each lot having a different type of functional group, and any other mixture, wherein the microparticles can all be administered at the same time or wherein different lots of microparticles can be administered at different time points.

14. Method of one of claims 11 to 13, wherein the method is a histological method or a fluorescence detection method; and/or wherein the orthogonal system is a click system.

15. Method of claim 14, wherein in step a) additionally an oxygen scavenging system is used; wherein optionally the oxygen scavenging system comprises glucose oxidase, ß-D-glucose and catalase.
